(19) Europäisches Patentamt
European Patent Office
Office européen des brevets

(11) **EP 2 599 473 A1**

## (12) EUROPEAN PATENT APPLICATION
published in accordance with Art. 153(4) EPC

(43) Date of publication:
**05.06.2013 Bulletin 2013/23**

(21) Application number: **11812223.3**

(22) Date of filing: **01.07.2011**

(51) Int Cl.:
*A61K 8/35* (2006.01)      *A61K 8/19* (2006.01)
*A61K 8/365* (2006.01)      *A61K 8/55* (2006.01)
*A61K 9/107* (2006.01)      *A61K 31/122* (2006.01)
*A61K 47/02* (2006.01)      *A61K 47/10* (2006.01)
*A61K 47/12* (2006.01)      *A61K 47/18* (2006.01)
*A61K 47/24* (2006.01)      *A61P 17/00* (2006.01)
*A61Q 19/00* (2006.01)

(86) International application number:
**PCT/JP2011/065228**

(87) International publication number:
**WO 2012/014630 (02.02.2012 Gazette 2012/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.07.2010 JP 2010169443**

(71) Applicant: **FUJIFILM Corporation
Tokyo 106-8620 (JP)**

(72) Inventor: **KUSUDA, Fumi
Ashigarakami-gun
Kanagawa 258-8577 (JP)**

(74) Representative: **Morpeth, Fraser Forrest
Fujifilm Imaging Colorants Limited
Intellectual Property Group
P.O. Box 42
Hexagon Tower
GB-Blackley, Manchester M9 8ZS (GB)**

(54) **ASTAXANTHIN-CONTAINING AQUEOUS COMPOSITION, COSMETIC PREPARATION, AND METHOD FOR SUPPRESSING DEGRADATION OF ASTAXANTHIN**

(57)      Disclosed are an aqueous cosmetic preparation including at least (A) astaxanthin, (B) 20 μg/L or more of iron ions, and (C) an iron chelating agent, and a method for suppressing the decomposition of astaxanthin in which (C) an iron chelating agent is included in an aqueous composition including at least (A) astaxanthin and (B) 20 μg/L or more of iron ions.

**Description**

Technical Field

The present invention relates to an astaxanthin-containing aqueous composition, a cosmetic preparation, and a method for suppressing the decomposition of astaxanthin.

Background Art

Astaxanthin is a naturally-derived carotenoid known as having functions, such as antioxidant effect and anti-inflammatory effect, and is added and used in foods, cosmetic products, pharmaceutical products, other processed goods, and the like. As an application example focusing on various functions of astaxanthin, for example, Japanese Patent Application Laid-Open (JP-A) No. 2000-136123 discloses a topical preparation for skin containing astaxanthin as an active oxygen-scavenging agent. JP-A No. 2002-128651 discloses a photo-aging inhibitor containing astaxanthin as a component having a singlet oxygen quenching ability. In addition, JP-A No. 9-143063 discloses a composition including a kind of astaxanthin and a medicinal agent(s).

Meanwhile, carotenoids, including astaxanthin and the like, have an unstable structure and thus decomposed by heat, light, oxygen, or the like, so that it has been difficult to maintain stability over time of carotenoids. Regarding methods for maintaining the stability of carotenoids, for example, JP-A No. 6-264055 discloses a method for adding an organic acid in a carotenoid-containing product. JP-A No. 2008-74717 discloses the inclusion of deoxygenated water and an iron chelating agent together with a carotenoid to suppress odors caused by an impurity contained in a carotenoid-containing emulsion composition and oxidation and improve stability over time.

SUMMARY OF THE INVENTION

Problem to Be Solved by Invention

According to a first aspect of the present invention, it is an object of the invention to provide an astaxanthin-containing aqueous composition having excellent stability over time of contained astaxanthin and a cosmetic preparation including the composition.
In addition, according to a second aspect of the present invention, it is an object of the invention to provide a method for suppressing the decomposition of astaxanthin.

Means for Solving the Problem

Specific means for solving the problems regarding the first aspect are as follows:

<1> An astaxanthin-containing aqueous composition including at least astaxanthin, 20 μg/L or more of iron ions, and an iron chelating agent.
<2> The astaxanthin-containing aqueous composition of <1>, further including dipropylene glycol.
<3> The astaxanthin-containing aqueous composition of <1> or <2>, wherein the iron chelating agent is at least one selected from the group consisting of citric acid or a salt thereof, ethylenediaminetetraacetic acid (EDTA) or a salt thereof, etidronic acid or a salt thereof, diethylenetriaminepentaacetic acid (DTPA or a salt thereof), hydroxyethylenediaminetriacetic acid (HEDTA) or a salt thereof, ethylenediamine-N,N, N', N'-tetramethylene phosphoric acid (EDTMP) or a salt thereof, and tartaric acid or a salt thereof.
<4> The astaxanthin-containing aqueous composition of any one of <1> to <3>, further including a water-soluble antioxidant.
<5> A cosmetic preparation including the astaxanthin-containing aqueous composition of any one of <1> to <4>.

Specific means for solving the problems regarding the second aspect are as follows:

<6> A method for suppressing the decomposition of astaxanthin, the method including: including an iron chelating agent in an aqueous composition including at least astaxanthin and 20 μg/L or more of iron ions.
<7> The method for suppressing the decomposition of astaxanthin of <6>, further including: including dipropylene glycol.
<8> The method for suppressing the decomposition of astaxanthin of <6> or <7>, wherein the iron chelating agent is at least one selected from the group consisting of citric acid or a salt thereof, ethylenediaminetetraacetic acid (EDTA), etidronic acid, diethylenetriaminepentaacetic acid (DTPA), hydroxyethylenediaminetriacetic acid (HEDTA), ethylenediamine-N,N, N', N'-tetramethylene phosphoric acid (EDTMP), and tartaric acid.
<9> The method for suppressing the decomposition of astaxanthin of any one of <6> to <8>, further including: including a water-soluble antioxidant in the aqueous composition. Advantageous Effect of Invention

According to the first aspect of the present invention, there can be provided an astaxanthin-containing aqueous composition having excellent stability over time of contained astaxanthin and a cosmetic preparation including the composition.
According to the second aspect of the present invention, there can be provided a method for suppressing the decomposition of astaxanthin.

EMBODIMENT FOR CARRYING OUT INVENTION

Hereinafter, detailed descriptions will be given of an astaxanthin-containing aqueous composition of the present invention, a cosmetic preparation including the composition, and a method for suppressing the decomposition of astaxanthin of the invention.

<Astaxanthin-Containing Aqueous Composition>

The astaxanthin-containing aqueous composition of the present invention (hereinafter may also be referred to as simply "aqueous composition") is characterized by including at least astaxanthin, 20 $\mu$g/L or more of iron ions, and an iron chelating agent.
The astaxanthin-containing aqueous composition of the present invention has the above configuration, thereby effectively suppressing the decomposition of astaxanthin contained in the composition and thus dramatically improving stability over time of astaxanthin. This is based on a finding obtained by the present inventor that, when a specific amount or more of iron ions coexists in an aqueous composition containing astaxanthin, the decomposition of astaxanthin is significantly promoted.
As one factor causing the mixing of iron ions in the aqueous composition, there may be mentioned the mixing of an iron component contained as an impurity included in a production raw material. For example, dipropylene glycol, which is a kind of polyhydric alcohol, is a component used in a raw material for a cosmetic preparation or the like, as a component for providing a moisturizing function, a viscosity control function, or the like. The component, however, often tends to contain iron ions as an impurity. In addition, iron ions may be mixed in the composition due to elution from a stainless steel pot or pipe in a production process, or the like.
Even when using a component highly likely to allow the mixing of iron ions in the composition, the present invention enables effective suppression of the decomposition of astaxanthin due to iron ions. Accordingly, various components can be selected without any concern about the decomposition of astaxanthin due to iron ions. This greatly improves the flexibility of design of the aqueous composition.
The astaxanthin-containing aqueous composition of the present invention is preferably an oil-in-water emulsion composition.
As used herein, the term "aqueous" in the present invention means a system containing water and/or a water-soluble solvent in an amount of at least 50% by mass.
In addition, in the present invention, any numerical range expressed herein using "to" refers to a range including the numerical values before and after the "to", as a minimum value and a maximum value, respectively.
Furthermore, in a case in which the amount of a component in the composition is indicated in the present invention, when there are plural substances corresponding to the component in the composition, the indicated amount means the total amount of the plural substances present in the composition, unless specifically stated otherwise.
Hereinafter, a description will be given of various components indispensably or optionally included in the aqueous composition of the present invention.

(1) Astaxanthin

The astaxanthin included in the aqueous composition of the present invention has antioxidant effect, anti-inflammatory effect, skin aging prevention effect, whitening effect, and the like and is known as a component capable of providing excellent emollient effect, anti-skin aging effect, and antioxidant effect.
In the present invention, the term "astaxanthin" encompasses both astaxanthin and derivatives thereof such as astaxanthin ester, (hereinafter may also be referred to as "astaxanthins" as needed).
The astaxanthin in the present invention may be any as long as it can be obtained by a usual method, besides those derived from natural materials such as plants, algae, and bacteria.
Examples of the natural materials include red yeast phaffia, green alga Haematococcus, marine bacteria, and krill. In addition, there may be mentioned extracts from extracts or the like obtained from culture products thereof.
The astaxanthins may be included in the aqueous composition of the present invention, as astaxanthin-containing oil obtained by separation and extraction (additionally, purification may be appropriately performed as needed) from natural materials containing astaxanthins.

Examples of the astaxanthin-containing oil include an extract from a culture product obtained by culture of red yeast phaffia, green alga Haematococcus, marine bacteria, or the like and an extract from Antarctic krill or the like.
An extract from Haematococcus algae (Haematococcus algae-derived coloring matter) is known to be different from a krill-derived coloring matter and synthesized astaxanthin in terms of the kind of ester and the content ratio thereof.

The astaxanthins in the present invention may be the above-mentioned extracts, those obtained by furthermore appropriately purifying the extracts as needed, or synthesized products.
The astaxanthins are particularly preferably an extract from Haematococcus algae (also called a Haematococcus algae extract) from the viewpoint of product quality and productivity.

Specific examples of the origin of the Haematococcus algae extract that may be used in the present invention include Haematococcus pluvialis, Haematococcus lacustris, Haematococcus capensis, Haematococcus droebakensis, and Haematococcus zimbabwiensis.

Furthermore, the present invention can use widely commercially available Haematococcus algae extracts, which can be obtained, for example, as ASTOTS-S, ASTOTS-2.5 O, ASTOTS-5 O, and ASTOTS-10 O (all of which are trade names) produced by Takeda Shiki Co., Ltd., AstaREAL Oil 50F and AstaREAL Oil 5F (both of which are trade names) produced by Fuji Chemical Industry Co. Ltd., and BioAstin SCE7 (trade name) produced by Toyo Koso Kagaku Co. Ltd.

The amount of astaxanthins as a coloring matter pure component in a Haematococcus algae extract that may be used in the present invention is preferably from 0.001 % by mass to 50% by mass, and more preferably from 0.01 % by mass to 25% by mass, from a viewpoint of handling of the aqueous composition upon production.

The Haematococcus algae extract that may be used in the present invention includes astaxanthin or an ester conjugate thereof as a coloring matter pure component similar to a coloring matter as described in JP-A No. 2-49091. The ester conjugate thereof is included in an amount of generally 50% by mole or more, preferably 75% by mole or more, and more preferably 90% by mole or more.
A more detailed description is given in "Astaxanthin-no-Kagaku (Chemistry of Astaxanthin)", 2005 on the Internet (URL: http://www.astaxanthin.co.jp/chemical/basic.htm).

(2) Iron Ions

The aqueous composition of the present invention includes 20 μg/L or more of iron ions.
When an astaxanthin-containing aqueous composition includes 20 μg/L or more of iron ions, the decomposition of astaxanthin significantly proceeds, and thus stability over time of the aqueous composition is reduced down to a practically problematic level. Particularly, inclusion of 50 μg/L or more of iron ions results in the progression of decomposition of astaxanthin down to a level where advantages obtained by the inclusion of astaxanthin are hardly obtainable. However, the aqueous composition of the present invention shows excellent stability over time even when the composition includes such an amount of iron ions.
There is no limitation to an upper limit value of the amount of iron ions included in the aqueous composition of the present invention. However, considering functions of other components included in the aqueous composition, the upper limit value of the amount thereof is 200 μg/L.

The amount of iron ions included in the aqueous composition of the present invention is a value measured by HR-ICP-MS (trade name: ELEMENT-XR, produced by Thermo Fisher Inc.).

(3) Iron Chelating Agent

The iron chelating agent included in the aqueous composition of the present invention may be any as long as it is a compound capable of forming a chelating bond with an iron ion.

Examples of the iron chelating agent include citric acid or a salt thereof, ethylenediaminetetraacetic acid (EDTA) or a salt thereof, etidronic acid or a salt thereof, diethylenetriaminepentaacetic acid (DTPA) or a salt thereof, hydroxyethylenediaminetriacetic acid (HEDTA) or a salt thereof, ethylenediamine-N,N, N', N'-tetramethylene phosphoric acid (EDTMP) a salt thereof, tartaric acid or a salt thereof, phytic acid or a salt thereof, pyrophosphoric acid or a salt thereof, polyphosphoric acid or a salt thereof, and metaphosphoric acid or a salt thereof. From a viewpoint of producing no precipitate due to complex salt, the iron chelating agent is preferably at least one selected from the group consisting of citric acid or a salt thereof, ethylenediaminetetraacetic acid (EDTA) or a salt thereof, etidronic acid or a salt thereof, diethylenetriaminepentaacetic acid (DTPA) or a salt thereof, hydroxyethylenediaminetriacetic acid (HEDTA) or a salt thereof, ethylenediamine-N,N, N', N'-tetramethylene phosphoric acid (EDTMP) a salt thereof, and tartaric acid or a salt thereof, more preferably citric acid or a salt thereof, ethylenediaminetetraacetic acid (EDTA) or a salt thereof, and etidronic acid or a salt thereof, and still more preferably etidronic acid or a salt thereof.

The iron chelating agent included in the aqueous composition may be one kind alone or two or more kinds.

The amount of the iron chelating agent included is, from a viewpoint of iron ion-sealing ability, preferably 0.5 to 10 times by mole, more preferably 0.7 to 5 times by mole, and still more preferably 1 to 3 times by mole, with respect to that of the iron ions present in the aqueous composition.

In addition, the amount of the included iron chelating agent with respect to a total mass of the aqueous composition is preferably in a range of from 0.001 % by mass to 10% by mass, more preferably in a range of from 0.01 % by mass to 3% by mass, and still more preferably in a range of from 0.1 % by mass to 1% by mass.

(4) Water-Soluble Antioxidant

The aqueous composition of the present invention preferably further includes a water-soluble antioxidant. Inclusion of a water-soluble antioxidant can further improve the stability over time of the aqueous composition.

As the water-soluble antioxidant, for example, a known water-soluble antioxidant may be used. Suitable examples of the water-soluble antioxidant include a compound group consisting of (I) ascorbic acid, ascorbic acid derivatives, or salts thereof and erythorbic acid, erythorbic acid derivatives, or salts thereof and (II) a compound group consisting of polyphenols.

(I) Ascorbic acid, ascorbic acid derivatives, or salts thereof and erythorbic acid, erythorbic acid derivatives, or salts thereof

Examples of ascorbic acid, ascorbic acid derivatives, or salts thereof include L-ascorbic acid, L-ascorbic acid sodium, L-ascorbic acid potassium, L-ascorbic acid calcium, L-ascorbic acid phosphoric ester, a magnesium salt of L-ascorbic acid phosphoric ester, L-ascorbic acid sulfuric ester, an L-ascorbic acid sulfuric ester 2-sodium salt, L-ascorbic acid stearic ester, L-ascorbic acid 2-glucoside, L-ascorbic acid palmitic ester, and L-ascorbyl tetraisopalmitate

Among them, a magnesium salt of L-ascorbic acid phosphoric ester is particularly preferable.

Examples of erythorbic acid, erythorbic acid derivatives, or salts thereof include erythorbic acid, erythorbic acid sodium, erythorbic acid potassium, erythorbic acid calcium, erythorbic acid phosphoric ester, and erythorbic acid sulfuric ester.

As the water-soluble antioxidant belonging to the compound group (I), a generally commercially available antioxidant may be used as needed. Examples of the commercially available antioxidant include L-ascorbic acid (available from Takeda Pharmaceutical Company Limited, Fuso Chemical Co., Ltd., BASF Japan Co., Ltd, Daiichi Pharmaceutical Co., Ltd., or the like), L-ascorbic acid sodium (available from Takeda Pharmaceutical Company Limited, Fuso Chemical Co., Ltd., BASF Japan Co., Ltd, Daiichi Pharmaceutical Co., Ltd., or the like), L-ascorbic acid 2-glucoside (Wako Pure Chemical Industries, Ltd., trade name: AA-2G (available from Hayashibara Biochemical Laboratories), L-ascorbic acid phosphate magnesium (trade name: ASCORBIC ACID PM "SDK" (available from Showa Denko K.K.); trade name: NIKKOL VC-PMG (Nikko Chemicals); and trade name: SEAMATE (available from Takeda Pharmaceutical Company Limited), and ascorbyl palmitate (trade name: DSM, available from Nutrition Japan Co., Ltd., Kongo Yakuhin Co., Ltd., Merck & Co., Inc., or the like).

(II) Compound group consisting of polyphenols

Examples of the compound group consisting of polyphenols include flavonoids (catechin, anthocyanin, flavone glycoside, isoflavone glycoside, flavan glycoside, flavanone, and rutin glycoside), phenolic acids (chlorogenic acid, ellagic acid, gallic acid, and propyl gallate), lignan glycosides, curcumin glycosides, and coumarins. In addition, these compounds are abundantly contained in extracts derived from natural materials and therefore can be utilized in a form of extract.

As the water-soluble antioxidant belonging to the compound group (II), a generally commercially available antioxidant may be used as needed. Examples of the commercially available antioxidant include ellagic acid (available from Wako Pure Chemical Industries, Ltd., or the like), rosemary extracts (trade name: RM-21A, RM-21E, available from Mitsubishi-Kagaku Foods Corporation or the like), catechin (trade name: SUNCATOL W-5, No. 1, available from Taiyo Chemical Co., Ltd., or the like), gallic acid sodium (trade name: SUNCATOL available from Taiyo Chemical Co., Ltd., or the like), rutin/glucosyl rutin/enzyme-degrading rutin (trade name: RUTIN K-2, P-10, available from Kiriya Chemical Co., Ltd., and trade name: αG RUTIN available from Hayashibara Biochemical Laboratories, or the like).

Among these water-soluble antioxidants, from a viewpoint of strong antioxidant ability, ascorbic acid or ascorbic acid derivatives are particularly preferable.

The amount of the water-soluble antioxidant included in the aqueous composition of the present invention is preferably from 0.1 % by mass to 6% by mass, more preferably from 0.5 % by mass to 5% by mass, and still more preferably from 1 % by mass to 3% by mass, with respect to the total mass of the aqueous composition.

(5) Polyhydric Alcohol

The aqueous composition of the present invention may further include a polyhydric alcohol.
The polyhydric alcohol has a moisturizing function, a viscosity controlling function, or the like.
In addition, in a case where the aqueous composition of the present invention is an emulsion composition, the polyhydric alcohol also serves to reduce interfacial tension between water and a component of oils and fats to facilitate the expansion of the interface, thereby allowing easy formation of minute and stable emulsion composition particles. Accordingly, the inclusion of a polyhydric alcohol in the aqueous composition of the present invention is preferable from the viewpoint that when the aqueous composition of the present invention is formed as an emulsion composition, the size of the emulsion composition particles becomes smaller, and the small particles are maintained stably for a long time.
Further, the addition of a polyhydric alcohol may lower water activity of the aqueous composition of the present invention, which can suppress the increase of microorganisms.
The polyhydric alcohol that may be included in the aqueous composition of the present invention is not specifically limited for use as long as it is a divalent or more alcohol.
Examples of the polyhydric alcohol include glycerin, diglycerin, triglycerin, polyglycerin, 3-methyl-1,3-butanediol, 1,3-butylene glycol, isoprene glycol, polyethylene glycol, 1,2-pentane diol, 1,2-hexane diol, propylene glycol, dipropylene glycol, polypropylene glycol, ethylene glycol, diethylene glycol, pentaerythritol, neopentyl glycol, maltitol, reduced sugar syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitan, trehalose, amylolytic sugar, and amylolytic sugar reduced alcohol. These may be used alone or in a form of mixture of a plurality of kinds thereof.
Among the above-mentioned polyhydric alcohols, dipropylene glycol tends to contain many iron ions as an impurity in the production process and facilitates the introduction of iron ions in the aqueous composition. The use of such a polyhydric alcohol causes the decomposition of astaxanthin. However, even when using such a polyhydric alcohol, the aqueous composition of the present invention suppresses the decomposition of astaxanthin to exhibit excellent stability over time of astaxanthin.
Furthermore, as the polyhydric alcohol, it is preferable to use a polyhydric polyalcohol having three or more hydroxyl groups in a single molecule thereof. Thereby, when the aqueous composition of the present invention is applied to an emulsion composition, the interfacial tension between an aqueous solvent and a component of oils and fats can be reduced more effectively, so that more minute and stable microparticles can be formed. As a result, for example, when the aqueous composition of the present invention is used in food products, intestinal absorbability can be further increased, and when used in transdermal drugs or cosmetic products, percutaneous absorbability can be further increased.
The amount of the polyhydric alcohol included in the aqueous composition of the present invention is preferably from 10 % by mass to 60 % by mass, preferably from 20 % by mass to 55% by mass, and still more preferably from 30 % by mass to 50% by mass. When the polyhydric alcohol content is 10% by mass or more, sufficient stability over time may be obtained regardless of the kind of the oil-soluble component, the content thereof, or the like. On the other hand, when the polyhydric alcohol content is 60% by mass or less, intended advantages may be obtained while controlling the viscosity of the emulsion composition composition in an appropriate range.

(6) Emulsifier

In a case where the aqueous composition of the present invention is formed as an oil-in-water emulsion composition that is a preferred embodiment thereof, the composition preferably includes an emulsifier.
The emulsifier is not specifically limited, but preferably a nonionic emulsifier. Examples of the nonionic emulsifier include glycerin fatty acid ester, organic acid monoglyceride, polyglycerin fatty acid ester, propylene glycol fatty acid ester, polyglycerin condensed ricinoleic acid ester, sorbitan fatty acid ester, and sucrose fatty acid ester. More preferable examples thereof are polyglycerin fatty acid ester, sorbitan fatty acid ester, and sucrose fatty acid ester. In addition, the emulsifier does not necessarily have to be one highly purified by distillation or the like and may be a reaction mixture product.
The polyglycerin fatty acid ester is an ester of a polyglycerin having an average polymerization degree of 2 or more, preferably from 6 to 15, and more preferably from 8 to 10 and a fatty acid having from 8 to 18 carbon atoms, such as caprylic acid, capric acid, lauric acid, myristic acid, palmitic acid, stearic acid, oleic acid, or linoleic acid. Preferable examples of the polyglycerin fatty acid ester include hexa-glycerin mono-oleic acid ester, hexa-glycerin mono-stearic acid ester, hexa-glycerin mono-palmitic acid ester, hexa-glycerin mono-myristic acid ester, hexa-glycerin mono-lauric acid ester, deca-glycerin mono-oleic acid ester, deca-glycerin mono-stearic acid ester, deca-glycerin mono-palmitic acid ester, deca-glycerin mono-myristic acid ester, and deca-glycerin mono-lauric acid ester. These polyglycerin fatty acid esters may be used alone or as a mixture thereof. Examples of commercially available polyglycerin fatty acid esters include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC, and NIKKOL Decaglyn PR-20 (all of which are trade names) produced by Nikko Chemicals Co., Ltd.; RYOTO Polygly Ester L-10D, L-7D, M-10D, M-7D, P-8D, S-28D, S-24D, SWA-20D, SWA-15D, SWA-10D, O-50D, O-15D, B-100D, B-70D, and ER-60D (all of which are trade names) produced by Mitsubishi-Kagaku Foods Corporation; SUNSOFT Q-17UL, SUNSOFT Q-14S, and SUNSOFT A-141C (all of which are trade names) produced by Taiyo Chemical Co., Ltd.; POEM DO-100 and POEM J-0021 (both of which are trade names) produced by Riken Vitamin Co., Ltd.

The sorbitan fatty acid ester is preferably one containing a fatty acid having 8 or more carbon atoms, and more preferably one containing a fatty acid having 12 or more carbon atoms. Preferable examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate. These sorbitan fatty acid esters may be used alone or as a mixture thereof. Examples of commercially available sorbitan fatty acid ester include NIKKOL SL-10, SP-10V, SS-10V, SS-10MV, SS-15V, SS-30V, SI-10RV, SI-15RV, SO-10V, SO-15MV, SO-15V, SO-30V, SO-10R, SO-15R, SO-30R, and SO-15EX (all of which are trade names) by produced by Nikko Chemicals Co., Ltd., and SOLGEN 30V, 40V, 50V, 90, and 110 (all of which are trade names) produced by Daiichi Kogyo Seiyaku Co., Ltd.

The sucrose fatty acid ester is preferably one containing a fatty acid having from 12 or more carbon atoms, and more preferably one containing a fatty acid having from 12 to 20 carbon atoms. Preferable examples of the sucrose fatty acid ester include sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmitic acid ester, sucrose monomyristic acid ester, and sucrose monolauric acid ester. In the present invention, these sucrose fatty acid esters may be used alone or as a mixture thereof. Examples of commercially available sucrose fatty acid esters include RYOTO Sugar Esters S-070, S-170, S-270, S-370, S-370F, S-570, S-770, S-970, S-1170, S-1170F, S-1570, S-1670, P-070, P-170, P-1570, P-1670, M-1695, O-170, O-1570, OWA-1570, L-195, L-595, L-1695, LWA-1570, B-370, B-370F, ER-190, ER-290, and POS-135 (all of which are trade names) produced by Mitsubishi-Kagaku Foods Corporation; and DK Esters SS, F160, F140, F110, F90, F70, F50, F-A50, F-20W, F-10, F-A10E, COSMELIKE B-30, S-10, S-50, S-70, S-110, S-160, S-190, SA-10, SA-50, P-10, P-160, M-160, L-10, L-50, L-160, L-150A, L-160A, R-10, R-20, O-10, and O-150 (all of which are trade names) produced by Daiichi Kogyo Seiyaku Co., Ltd.

The added amount of the emulsifiers as mentioned above is preferably from 0.1 % by mass to 50% by mass, more preferably from 0.5 % by mass to 20% by mass, and still more preferably from % by mass to 15% by mass.

In addition, as another example of the emulsifier, lecithin is also effective. Lecithin contains a glycerin skeleton, a fatty acid residue, and a phosphoric acid residue as essential constituent components, to which a base, a polyhydric alcohol, and the like are bonded, and is also called phospholipid. Lecithin has a hydrophilic group and a hydrophobic group in the molecule thereof, and thus, is widely used as an emulsifier in the fields of foods, pharmaceutical products, and cosmetic products.

Industrially used lecithin has a lecithin purity of 60% or more, and such a lecithin can be used also in the present invention. A preferable lecithin is one generally called a high-purity lecithin, which has preferably a purity of 80% or more, and more preferably 90% or more. The lecithin purity is obtained by subtracting the weights of a toluene-insoluble substance and an acetone-soluble substance using the properties of lecithin in which it is easily soluble in toluene and insoluble in acetone.

As lecithin, there may be mentioned various conventionally known products extracted and separated from living bodies of plants, animals, and microorganisms. Specific examples of such a lecithin include various kinds of lecithins derived from plants such as soybean, corn, peanut, rapeseed, or wheat; egg yolk; animals such as cattle; and microorganisms such as Escherichia coli. Examples of compounds representing such a lecithin include glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, and diphosphatidylglycerin (cardiolipin); and sphingolecithins such as sphingomyelin.

In addition, in the present invention, besides the high-purity lecithin as mentioned above, there may be used a hydrogenated lecithin, an enzyme-decomposed lecithin, an enzyme-decomposed hydrogenated lecithin, a hydroxylecithin, or the like. These lecithins may be used alone or in a form of mixture of a plurality of kinds thereof.

When using lecithin, the amount of added lecithin is preferably from 0.1 %by mass to 10% by mass, more preferably form 0.2%by mass to 5% by mass, and still more preferably from 0.5 % by mass to 2% by mass, with respect to the total mass of the composition.

(7) Other Oil Components

The aqueous composition of the present invention may include various oil components other than the above-described astaxanthin.
An oil component that can be used in the present invention is not specifically limited as long as it is a component that is insoluble or hardly insoluble in an aqueous medium, particularly in water, and soluble in an oil medium. The phrase "insoluble in an aqueous medium" means that a solubility in 100 mL of an aqueous medium is 0.01 g or less at 25°C, and the phrase "hardly soluble in an aqueous medium" means that a solubility in 100 mL of an aqueous medium is 0.01 g or more and 0.1 g or less at 25°C. Furthermore, the term "functional component" in the present specification means a component that can be expected to induce a predetermined physiological effect in a living body to which the component is applied.

Examples of these oil components include a wide range of oil components showing useful effects when used in cosmetic products. From the aspect of chemical structure, there may be mentioned fats and oils, hydrocarbons, waxes, esters, higher alcohols, high polymers, oil-soluble coloring matters, oil-soluble proteins, and the like. In addition, as mixtures thereof, the examples thereof include various vegetable oils and animal oils.

Examples of these oil components include oils such as coconut oil, olive oil, corn oil, and jojoba oil; higher alcohols such as behenyl alcohol, stearyl alcohol, and cetanol; sterols such as cholesterol and phytosterol; esters such as ethylhexyl palmitate, isopropyl myristate, and octyldodecyl myristate; and hydrocarbons such as squalane, hydrogenated polydecene, and hydrogenated polyisobutene.

In addition, as functional oil components having distinctive functions, there may also be included carotenoids such as beta carotene, zeaxanthin, lycopene, and lutein, vitamins E such as tocopherol and tocotrienol, ubiquinones such as coenzyme Q10, and omega-3 oils such as EPA, DHA, and linolenic acid.

Furthermore, there may also be included active ceramides such as ceramide I, ceramide II, ceramide III, ceramide V, and ceramide VI, glycosphingolipids such as glucosylceramide and galactosylceramide; sphingomyelins; and pseudo-ceramides, although they are expensive as oil components having moisturizing function.

In the aqueous composition of the present invention, the amount of the other oil component included is preferably from 0.1 % by mass to 50% by mass, more preferably from 0.2 % by mass to 25% by mass, and still more preferably from 0.5 % by mass to 10% by mass, with respect to the total mass of the composition. The oil component content of 0.1% by mass or more as above is preferable since efficacy of the active component can be sufficiently exhibited, thus facilitating application of the aqueous composition of the present invention to a cosmetic preparation. On the other hand, the content of 50% by mass or less is preferable since increase in the size of dispersed particles and deterioration of emulsification stability are suppressed and thus a stable composition can be obtained.

<Method for Suppressing Decomposition of Astaxanthin>

The method for suppressing the decomposition of astaxanthin according to the present invention is a method for suppressing the decomposition of astaxanthin applied to an astaxanthin-containing aqueous composition, and characterized in that an iron chelating agent is included in an aqueous composition including at least astaxanthin and 20 $\mu$g/L or more of iron ions.

Hereinafter, the method for suppressing the decomposition of astaxanthin of the present invention will be described based on a method for producing the aqueous composition of the present invention as an emulsion composition that is a preferable form thereof.

The aqueous composition production method to which the decomposition suppression method of the present invention is applied is not specifically limited, but preferably has a process of forming an emulsion composition by mixing at least astaxanthin and an iron chelating agent. For example, preferably, the method includes the respective processes of: a) dissolving an iron chelating agent, a water-soluble emulsifier, a water-soluble antioxidant, and a polyhydric alcohol in an aqueous medium to obtain a water phase, b) mixing and dissolving astaxanthin, an oil-soluble emulsifier, an oil-soluble antioxidant, and if needed, other oil(s) and fat(s) to obtain an oil phase, and c) mixing together the water phase and the oil phase under stirring for emulsification dispersion to obtain an emulsion composition.

In addition, a high-concentration astaxanthin-containing emulsion composition including no iron chelating agent may be once produced, then, an iron chelating agent may be added to the composition, and the resulting composition may be diluted with an aqueous solvent such as water to prepare the aqueous composition of the present invention. Similarly, a high-concentration astaxanthin-containing emulsion composition including an iron chelating agent may be once produced, and then, without or with the addition of an iron chelating agent, the composition may be diluted with an aqueous solvent such as water to prepare the aqueous composition of the present invention.
Alternatively, after preparing once a high-concentration astaxanthin-containing emulsion composition and diluting it with an aqueous solvent such as water, and then, an aqueous components such as an iron chelating agent, a polyhydric alcohol, or a water-soluble antioxidant may be added thereto.

When employing those methods, a dilution ratio of the high-concentration astaxanthin-containing emulsion composition can be selected as needed, and is preferably 10 times or more and 200 times or less, more preferably 20 times or more and 150 times or less, and still more preferably 40 times or more and 100 times or less.

In the emulsification dispersion, it is particularly preferable to use two or more kinds of emulsification apparatuses in combination. For example, after performing emulsification using an ordinary emulsification apparatus utilizing shearing action of a stirrer, an impeller stirrer, a homomixer, or a continuous flow shearing apparatus, the emulsion may be passed through a high-pressure homogenizer or the like. The use of a high-pressure homogenizer allows the emulsion to have liquid droplets of more uniform microparticles. In addition, the emulsification dispersion may be performed a plurality of times to obtain liquid droplets of still more uniform particle size.

As the high-pressure homogenizer, there may be mentioned a chamber-type high-pressure homogenizer having a chamber with a fixed flow path of treatment liquid and a homogenous valve-type high-pressure homogenizer having a homogenous valve. Of these homogenizers, the homogenous valve-type high-pressure homogenizer can easily control the width of the flow path of treatment liquid, so that pressure and flow rate in operation can be arbitrarily set, and the operation range thereof is wide and thus widely used in the emulsification fields of particularly foods, cosmetic products, and the like. On the other hand, the chamber-type high-pressure homogenizer is used for purposes requiring ultra-high pressure since a pressure increasing mechanism is easily made, although its operational flexibility is low.

Examples of the chamber-type high-pressure homogenizer include MICROFLUIDIZER (trade name, produced by Microfluidics Corp.), NANOMIZER (trade name, produced by Yoshida Kikai Co., Ltd.), and ALTIMIZER (trade name, produced by Sugino Machine Limited).

Examples of the homogenous valve-type high-pressure homogenizer include GAULIN-TYPE HOMOGENIZER (trade name, produced by APV Co. Ltd.), RANNIE-TYPE HOMOGENIZER (trade name, produced by Rannie Co. Ltd.), HIGH-PRESSURE HOMOGENIZER (trade name, produced by Niro Soavi Co., Ltd.), HOMOGENIZER (trade name, produced by Sanwa Engineering Ltd.), HIGH-PRESSURE HOMOGENIZER (trade name, produced by Izumi Food Machinery Co., Ltd.), and ULTRA-HIGH-PRESSURE HOMOGENIZER (trade name, produced by Ika Co., Ltd.).

Dispersion by the high-pressure homogenizer seems to be caused by a large shearing force occurring when liquid passes through an extremely narrow (small) space. The magnitude of the shearing force is approximately proportional to pressure. As pressure becomes higher, the shearing force applied to particles dispersed in the liquid, namely a dispersion force, becomes stronger. However, since the majority of kinetic energy occurring when the liquid flows at high speed is converted to heat, as the pressure becomes higher, liquid temperature increases. This can promote the deterioration of a component(s) of the dispersion liquid and particle reaggregation. Accordingly, although the pressure of the high-pressure homogenizer has an optimum point, the optimum point seems to vary depending on the dispersed substance and the intended particle size. In the present invention, the pressure of the homogenizer is preferably 50 MPa or higher, more preferably from 50 MPa to 250 MPa, and still more preferably from 100 MPa to 250 MPa for treatment. In addition, the emulsion is preferably cooled down through any given cooler within 30 seconds and preferably within 3 seconds immediately after passing through the chamber.

As another effective method for obtaining a minute emulsion, there may be mentioned the use of a supersonic homogenizer. Examples of the supersonic homogenizer include supersonic homogenizers: US-1200T, RUS-1200T, and MUS-1200T (all of which are trade names, produced by Nihonseiki Kaisha Ltd.,) and ultrasonic processers: UIP 2000, UIP-4000, UIP-8000, and UIP-16000 (all of which are trade names, produced by D. Hielscher GmbH). In the present invention, using these ultrasonic homogenizers, minute emulsification may be performed at a frequency of 25 kHz or less and preferably 15 to 20 kHz, and at an energy density of a dispersion portion of 100 $W/cm^2$ or more and preferably 120 $W/cm^2$.

Ultrasonic irradiation may be performed in a batch mode. In that case, it is preferable to combine with a means for stirring the entire dispersion liquid. As the combined stirring means, there may be used stirring by an agitator, a magnetic stirrer, a disper, or the like. More preferably, flow-system ultrasonic irradiation may be performed. The flow system is namely a system provided with a dispersion liquid supply tank and a supply pump to feed a dispersion liquid at a constant flow rate into a chamber equipped with an ultrasonic irradiation section. The supply of the liquid into the chamber is effective in any direction, but particularly preferable is a method supplying the liquid in a direction in which the flow of the liquid collides vertically with an ultrasonic irradiation surface.

Time for ultrasonic irradiation is not specifically limited, but preferably, 2 to 200 minutes/kg as time during which ultrasonic irradiation is substantially being performed in the vessel. If the time therefor is too short, emulsification is insufficient, whereas if it is too long, reaggregation can occur. An optimum time for ultrasonic irradiation varies with the emulsion. The optimum time therefor is generally preferably from 10 minutes to 100 minutes.

Due to temperature increase of the emulsion by ultrasonic irradiation at high-energy density, the deterioration of constituent components and the reaggregation of particles in the emulsion can occur. Accordingly, preferably, a cooling means is used together. In the case of batch irradiation, an irradiation vessel may be cooled from outside or a cooling unit may be arranged in the vessel. In addition, in the case of the flow system, besides cooling of the ultrasonic irradiation chamber from outside, it is preferable to arrange a cooling means such as a heat exchanger in a route of flow circulation.

By combining an ultrasonic homogenizer with an ultra high-pressure homogenizer, dispersion can be more preferably performed. Specifically, performing emulsification with an ordinary emulsification apparatus utilizing shearing action and then performing ultra high-pressure homogenizer dispersion increase efficiency of the ultra high-pressure homogenizer dispersion and thus can lead to reduction of the number of passes, as well as reduction of coarse particles enables production of a high-quality emulsion. In addition, by further performing ultrasonic irradiation after the ultra high-pressure homogenizer emulsification, coarse particles can be reduced. Furthermore, ultra high-pressure dispersion and ultrasonic irradiation may be repeated in an arbitrary order, such as performing these steps alternately.

<Purposes of Use>

The aqueous composition of the present invention has not only various excellent functionalities due to astaxanthin but also excellent stability over time. Therefore, the aqueous composition of the invention is preferably used as it is or as a component material, for various purposes of uses corresponding to the functions of astaxanthin and other component(s) included in the composition.

Examples of such purposes of uses include a wide range of pharmaceutical products (topical medication, drugs applied to the skin), cosmetic preparations, and foods. As used herein, the pharmaceutical products may be non-oral drugs such as suppositories and ointments (topical products for skin diseases), and the cosmetic preparations may be of any form of general form known as an aqueous cosmetic preparation, such as lotion, beauty lotion, gel, milky lotion, or cream..

When the astaxanthin-containing dispersion product of the present invention is used in a tropical product for skin disease and a cosmetic product, components that can be added to pharmaceutical products and cosmetic products may be appropriately added as needed.

EXAMPLES

Hereinafter, the present invention will be described in still more detail with reference to Examples. The present invention is, however, not limited to the Examples below as long as they do not depart from the gist of the invention.

(Examples 1 to 7 and Comparative Examples 1 to 6)

The following components were dissolved by heating at 70°C for one hour to obtain a water phase composition.

· Sucrose oleate: 13 g
· Decaglyceryl monooleate (HLB = 12): 25 g
· Glycerin: 500 g
· Pure water: 332 g

<Preparation of Oil Phase Composition>

In addition, the following components were dissolved by heating at 70°C for one hour to obtain an oil phase composition.

· Haematococcus algae extract (content of astaxanthins: 20% by mass): 40 g
· Lecithin (derived from soybeans): 90 g

The water phase was stirred by a homogenizer (at 10000 rpm) while maintaining at 70°C, and the oil phase obtained above was added thereto to obtain an emulsion. The obtained emulsion was subjected to high-pressure emulsification at a pressure of 200 MPa using ALTIMIZER HJP-25005 (trade name, produced by Sugino Machine Co. Ltd.). After that, the emulsion was filtered through a microfilter having an average hole diameter of 1 μm to prepare an astaxanthin-containing emulsion composition K-01.

Details of respective components used in the water phase composition and the oil phase composition are as follows:

The sucrose oleate used was RYOTO Sugar Ester O-1670 (trade name; HLB = 15) produced by Mitsubishi-Kagaku Foods Corporation, and the decaglyceryl monooleate used was NIKKOL Decaglyn 1-O (trade name; HLB = 12) produced by Nikko Chemicals Co., Ltd. The Haematococcus algae extract used was ASTOTS-S produced by Takeda Shiki Co., Ltd. The lecithin (derived from soybeans) used was LECION P (trade name) produced by Riken Vitamin Co., Ltd.

<Dilution>

In Comparative Example 1, 0.05 g of the emulsion composition K-01 was added into pure water for dilution to adjust the total amount of the composition to 100 g.

In Comparative Examples 2 to 4, after 0.05 g of the emulsion composition K-01 was added into 90 mL of pure water for dilution, iron (II) chloride tetrahydrate was added thereinto up to an amount of iron ions shown in Table 1, and additionally pure water was added to adjust the total amount of the composition to 100 g.

In Comparative Examples 5 and 6, after adding 0.05 g of the emulsion composition K-01 into 90 mL of pure water for dilution, iron (III) chloride was added in an amount necessary to reach an amount of iron ions shown in Table 1, as well as an iron chelating agent, propylene glycol (shown as DPG in Table 1), and L-ascorbic acid phosphate magnesium (shown as APM in Table 1) shown in Table 1 were added up to contained amounts shown in Table 1. Furthermore, pure water was added to adjust the total amount of the composition to 100 g.

In Examples 1 to 7, 0.05 g of the emulsion composition K-01 was added into pure water for dilution, and then, additionally, iron (II) chloride tetrahydrate with an amount necessary to reach an amount of iron ions shown in Table 1 and other components shown in Table 1 were added up to contained amounts shown in Table 1 to adjust the total amount of the composition to 100 g.

As a result of the above, compositions 1 to 7 of Examples 1 to 7 and compositions 1C to 6C of Comparative Examples 1 to 6 were prepared.

Details of the respective components shown in Table 1 are as follows:

Iron (II) chloride tetrahydrate used was iron (II) chloride tetrahydrate produced by Wako Pure Chemical Industries, Ltd.; citric acid used was citric acid produced by Wako Pure Chemical Industries, Ltd.; EDTA-2Na used was disodium edentate hydrate "exclusive for manufacturing purposes" produced by Wako Pure Chemical Industries, Ltd.; and sodium etidronate used was "CHELEST PH-210" (trade name) produced by Chelest Co. Ltd. In addition, dipropylene glycol (DPG) used was DPG-RF produced by ADEKA Corporation, and magnesium L-ascorbyl phosphate (APM) used was ascorbic acid "PM" (trade name) produced by Showa Denko K.K.

The amount of iron ions in each composition was measured by the above-mentioned method.

<Evaluation>

1. Evaluation of Stability Over Time (1)

In evaluation of stability over time (1), regarding the compositions 1 to 7 obtained in Examples 1 to 7 and the compositions 1C to 6C obtained in Comparative Examples 1 to 6, absorbance before and after preservation at high temperature (50°C) was measured to evaluate the stability over time of astaxanthin.

Specifically, there was prepared each lidded glass bottle (SV-15 produced by Nichiden-Rika Glass Co., Ltd.) containing 15 mL of each of the compositions 1 to 7 and the compositions 1C to 6C. Next, the respective lidded glass bottles were preserved in a thermostat chamber maintained at high temperature (50°C) for 6 days.

Each composition before and after the preservation was put in a 10 mm cell to measure absorbance at a wavelength of 478 nm using a UV-VIBLE spectrophotometer UV-2550 (trade name, produced by Shimadzu Corporation) and calculate an absorbance change rate (%) before and after the preservation by the following formula (1). The obtained absorbance change rate was used as an index of stability over time (1).

$$\text{Absorbance change rate (\%)} = \text{absorbance after preservation/absorbance before preservation} \times 100 \quad \text{Formula (1)}$$

Larger values of the absorbance change rate (%) show that the decomposition of astaxanthin under the high temperature environment is further suppressed and the stability over time thereof is better.

Table 1 shows the results.

2. Evaluation of Stability Over Time (2)

In evaluation of stability over time (2), regarding the compositions 1 to 7 obtained in Examples 1 to 7 and the compositions 1C to 6C obtained in Comparative Examples 1 to 6, absorbance before and after preservation at room temperature (23°C) was measured to evaluate the stability over time of astaxanthin.

Specifically, lidded glass bottles containing the compositions 1 to 7 and the compositions 1C to 6C were preserved in the same manner as in the evaluation of stability over time (1) except that the preservation condition of the lidded glass bottles in the evaluation of stability over time (1) was changed to a 3-month preservation in a thermostat chamber maintained at room temperature (23°C). Absorbance of each of the compositions before and after the preservation was measured to calculate an absorbance change rate (%). The obtained absorbance change rate (%) was used as an index of stability over time (2).

Larger values of the absorbance change rate (%) show that the decomposition of astaxanthin under room temperature is further suppressed and the stability over time thereof is better.

Table 1 shows the results.

In both the evaluations of stability over time (1) and (2), when the absorbance change rate (%) used as the index for the evaluation of stability over time is less than 80%, it shows that the degree of decomposition of astaxanthin is at a practically problematic level.

Table 1

| | Composition No. | Amount of iron ions (μg/L) | Emulsion composition K-01 (% by mass) | Iron chelating agent | | | DPG (% by mass) | AMP (% by mass) | Evaluation of stability over time | |
|---|---|---|---|---|---|---|---|---|---|---|
| | | | | Citric acid (% by mass) | EDTA-2Na (% by mass) | Etidronate disodium (% by mass) | | | Evaluation (1) high temperature (50°C) | Evaluation (2) room temperature (23°C) |
| Comparative Example 1 | C1 | 0.24 | 0.05 | 0 | 0 | 0 | 0 | 0 | 91% | 93% |
| Comparative Example 2 | C2 | 2.4 | 0.05 | 0 | 0 | 0 | 0 | 0 | 88% | 90% |
| Comparative Example 3 | C3 | 24 | 0.05 | 0 | 0 | 0 | 0 | 0 | 73% | 80% |
| Comparative Example 4 | C4 | 100 | 0.05 | 0 | 0 | 0 | 0 | 0 | 5% | 55% |
| Example 1 | 1 | 24 | 0.05 | 1 | 0 | 0 | 0 | 0 | 90% | 99% |
| Example 2 | 2 | 24 | 0.05 | 0 | 1 | 0 | 0 | 0 | 88% | 100% |
| Example 3 | 3 | 24 | 0.05 | 0 | 0 | 1 | 0 | 0 | 97% | 98% |
| Comparative Example 5 | C5 | 61 | 0.05 | 0 | 0 | 0 | 3 | 0 | 10% | 40% |
| Example 4 | 4 | 61 | 0.05 | 1 | 0 | 0 | 3 | 0 | 85% | 99% |
| Example 5 | 5 | 61 | 0.05 | 0 | 1 | 0 | 3 | 0 | 93% | 98% |
| Example 6 | 6 | 61 | 0.05 | 0 | 0 | 1 | 3 | 0 | 92% | 98% |
| Comparative Example 6 | C6 | 61 | 0.05 | 0 | 0 | 0 | 3 | 1 | 90% | 92% |
| Example 7 | 7 | 61 | 0.05 | 1 | 0 | 0 | 3 | 1 | 99% | 99% |

From the results shown in Table 1, the following conclusions can be made.

From the comparison between the composition C1 containing no iron (II) chloride tetrahydrate and the compositions C2 to C4 containing iron (II) chloride tetrahydrate, which are the compositions of the Comparative Examples, it was confirmed that, as the amount of iron ions contained in the composition increased, the absorbance change rate (%) reduced, and the presence of iron ions deteriorated the stability over time of astaxanthin.

Particularly, in the compositions C3 and C4 including 20 $\mu$g/L or more of iron ions, it is shown that stability over time was significantly deteriorated down to a practically problematic level.

On the other hand, it is found that the compositions 1 to 7, which are the compositions of the Examples, have good stability over time, which is not practically problematic, whether preserved under the environment of room temperature (23°C) or the environment of a high temperature (50°C), regardless of the amount of iron ions contained in the compositions. More specifically, the following was confirmed.

In the comparison between the compositions 1 to 3 containing an iron chelating agent and the composition C3 having the same formulation as that thereof except for containing no iron chelating agent, the compositions 1 to 3, which are those of the Examples, have better stability over time although all of them contain iron ions.

The composition C5 containing dipropylene glycol shows significant deterioration in the stability of astaxanthin due to the increase of iron ions, which seems to be derived from the dipropylene glycol. On the other hand, the compositions 4 to 6 of Examples 4 to 6 containing an iron chelating agent exhibit extremely good stability over time although they contain the same amount of iron ions as that in the composition C5 due to contained dipropylene glycol.

In addition, the composition 7 of Example 7 containing both an iron chelating agent and a water-soluble antioxidant exhibits extremely good stability over time when compared with the composition C5 of Comparative Example 5 containing neither an iron chelating agent nor a water-soluble antioxidant.

Furthermore, the composition 7 also has better stability over time when compared with the composition C6 of Comparative Example 6 containing no iron chelating agent.

The disclosure of Japanese Patent Application No. 2010-169443 is incorporated herein by reference. All documents, patent applications, and technical standards described in the present specification are incorporated herein by reference to the same extent as if each individual document, patent application, or technical standard were specifically and individually indicated to be incorporated by reference.

**Claims**

1. An astaxanthin-containing aqueous composition comprising at least astaxanthin, 20 $\mu$g/L or more of iron ions, and an iron chelating agent.

2. The astaxanthin-containing aqueous composition of claim 1, further comprising dipropylene glycol.

3. The astaxanthin-containing aqueous composition of claim 1 or 2, wherein the iron chelating agent is at least one selected from the group consisting of citric acid or a salt thereof, ethylenediaminetetraacetic acid (EDTA) or a salt thereof, etidronic acid or a salt thereof, diethylenetriaminepentaacetic acid (DTPA) or a salt thereof, hydroxyethylenediaminetriacetic acid (HEDTA) or a salt thereof, ethylenediamine-N,N, N', N'-tetramethylene phosphoric acid (EDTMP) or a salt thereof, and tartaric acid or a salt thereof.

4. The astaxanthin-containing aqueous composition of any one of claims 1 to 3, further comprising a water-soluble antioxidant.

5. A cosmetic preparation comprising the astaxanthin-containing aqueous composition of any one of claims 1 to 4.

6. A method for suppressing the decomposition of astaxanthin, the method comprising: including an iron chelating agent in an aqueous composition comprising at least astaxanthin and 20 $\mu$g/L or more of iron ions.

7. The method for suppressing the decomposition of astaxanthin of claim 6, further comprising: including dipropylene glycol.

8. The method for suppressing the decomposition of astaxanthin of claim 6 or 7, wherein the iron chelating agent is at least one selected from the group consisting of citric acid or a salt thereof, ethylenediaminetetraacetic acid (EDTA) or a salt thereof, etidronic acid or a salt thereof, diethylenetriaminepentaacetic acid (DTPA) or a salt thereof, hydroxyethylenediaminetriacetic acid (HEDTA) or a salt thereof, ethylenediamine-N,N, N', N'-tetramethylene phosphoric acid (EDTMP) or a salt thereof, and tartaric acid or a salt thereof.

**9.** The method for suppressing the decomposition of astaxanthin of any one of claims 6 to 8, further comprising: including a water-soluble antioxidant in the aqueous composition.

| **INTERNATIONAL SEARCH REPORT** | International application No. |
|---|---|
| | PCT/JP2011/065228 |

**A. CLASSIFICATION OF SUBJECT MATTER**
See extra sheet.

According to International Patent Classification (IPC) or to both national classification and IPC

**B. FIELDS SEARCHED**

Minimum documentation searched (classification system followed by classification symbols)
A61K8/35, A61K8/19, A61K8/365, A61K8/55, A61K9/107, A61K31/122, A61K47/02,
A61K47/10, A61K47/12, A61K47/18, A61K47/24, A61P17/00, A61Q19/00

Documentation searched other than minimum documentation to the extent that such documents are included in the fields searched
Jitsuyo Shinan Koho         1922-1996   Jitsuyo Shinan Toroku Koho   1996-2011
Kokai Jitsuyo Shinan Koho   1971-2011   Toroku Jitsuyo Shinan Koho   1994-2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2008-239609 A  (Kose Corp.),<br>09 October 2008 (09.10.2008),<br>example 4<br>(Family: none) | 1-9 |
| A | JP 2008-179619 A  (Nisshin Pharma Inc.),<br>07 August 2008 (07.08.2008),<br>paragraph [0036]<br>(Family: none) | 1-9 |
| A | WO 2009/048120 A1  (Nisshin Pharma Inc.),<br>16 April 2009 (16.04.2009),<br>paragraph [0038]<br>(Family: none) | 1-9 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 September, 2011 (21.09.11) | 04 October, 2011 (04.10.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/065228

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2000-136123 A  (Kose Corp.),<br>16 May 2000 (16.05.2000),<br>claims 1, 2, 4; paragraphs [0006] to [0009]<br>(Family: none) | 1-9 |
| A | JP 2-15017 A  (Kansai Paint Co., Ltd.),<br>18 January 1990 (18.01.1990),<br>page 1, right column, lines 6 to 12<br>(Family: none) | 1-9 |
| A | JP 2005-187329 A  (Lion Corp.),<br>14 July 2005 (14.07.2005),<br>paragraph [0012]<br>(Family: none) | 1-9 |
| A | JP 2000-342657 A  (Taisho Pharmaceutical Co.,<br>Ltd.),<br>12 December 2000 (12.12.2000),<br>paragraph [0004]<br>(Family: none) | 1-9 |

Form PCT/ISA/210 (continuation of second sheet) (July 2009)

| INTERNATIONAL SEARCH REPORT | International application No. |
|---|---|
| | PCT/JP2011/065228 |

Continuation of A. CLASSIFICATION OF SUBJECT MATTER
 (International Patent Classification (IPC))

*A61K8/35*(2006.01)i, *A61K8/19*(2006.01)i, *A61K8/365*(2006.01)i,
*A61K8/55*(2006.01)i, *A61K9/107*(2006.01)i, *A61K31/122*(2006.01)i,
*A61K47/02*(2006.01)i, *A61K47/10*(2006.01)i, *A61K47/12*(2006.01)i,
*A61K47/18*(2006.01)i, *A61K47/24*(2006.01)i, *A61P17/00*(2006.01)i,
*A61Q19/00*(2006.01)i

  (According to International Patent Classification (IPC) or to both national
  classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000136123 A
- JP 2002128651 A
- JP 9143063 A
- JP 6264055 A

- JP 2008074717 A
- JP 2049091 A
- JP 2010169443 A

**Non-patent literature cited in the description**

- *Astaxanthin-no-Kagaku,* 2005, http://www.astaxan-thin.co.jp/chemical/basic.htm